# EUROPÄISCHE PATENTANMELDUNG

(11) **EP 2 295 101 A2**
(43) Veröffentlichungstag der Anmeldung: **16.03.2011**
(21) Anmeldenummer: 10185080.8
(22) Anmeldetag: 29.01.2002
(51) Int. Cl.: A61M 16/00

(54) **Vorrichtung zur Zufuhr eines Atemgases**

(30) Priorität: 29.01.2001 DE 10103810
(62) Teilanmeldung aus: 02710048.6
(71) Anmelder: MAP Medizin-Technologie GmbH, 82152 Martinsried (DE)
(72) Erfinder: Schneider, Hartmut, Lutherville, MD 21093 (US); Meier, Jörg, 81547 München (DE); Heidmann, Dieter, Castle Hill, NSW 2154 (AU); Vögele, Harald, 82131 Gauting (DE); Madaus, Stefan, 82166 Gräfelfing (DE); Jakobs, Rainer, 81539 München (DE); Schätzl, Stefan, 82362 Weilheim (DE); Brandmeier, Richard, 86492 Egling (DE)
(74) Vertreter: Vossius & Partner

(57) **Zusammenfassung**

An apparatus for detecting an analyte in a sample of material includes a chamber for receiving an eluted sample of material from a sample acquisition device and a testing device adapted to detect the analyte. At least a part of the apparatus assembly is moveable between a sample preparation orientation and a testing orientation.

## Beschreibung

Die Erfindung betrifft eine Vorrichtung zur Zufuhr eines Atemgases zu einem Patienten im Rahmen der Diagnose und/oder der Therapie schlafbezogener Atmungsstörungen. Insbesondere betrifft die Erfindung ein CPAP-Gerät mit selbstabstimmender Bilevel-Druckregelung.

Schlafbezogene Atmungsstörungen, insbesondere in Verbindung mit Obstruktionen im Bereich der oberen Atemwege können auf physiologisch gut verträgliche Weise durch eine Überdruckbeatmung mit einem ggf. alternierenden, jedoch dauerhaft über dem Umgebungsdruckniveau liegenden Atemgasdruck therapiert werden. Diese allgemein als CPAP-Therapie bezeichnete Überdruckbeatmung basiert auf einer durch den Überdruck erreichten "pneumatischen Schienung" der oberen Atemwege. Durch diese pneumatische Schonung wird etwaigen Obstruktionen im Bereich der oberen Atemwege vorgebeugt. Um eine möglichst hohe physiologische Verträglichkeit der CPAP-Therapie zu gewährleisten, ist man bestrebt, den Beatmungsdruck möglichst gering zu halten und insbesondere während einer Expirationsphase gegenüber der inspirationsphasa abzusenken.

Günstige Beatmungsdruckpegel können bislang entweder durch den Patienten selbst gewählt werden oder beispielsweise auch unter ärztlicher Betreuung im Rahmen des Aufenthalts des Patienten in einem Schlafiabor ermittelt werden. Untersuchungen haben jedoch gezeigt, daß sowohl die vom Patienten selbst gewünschten, als auch die im Rahmen des Aufenthalts in einem Schlaflabor ermittelten Therspiedrücke für den Inspirationsdruck sowie für den üblichemeise etwas geringeren Expirationsciruck teilweise deutlich über den für den überwiegenden Tell der Schlafphase erforderlichen inspirations- und Expirationsdruckpegein liegen.

Unter dem Eindruck dieses Problems liegt der Erfindung liegt die Aufgabe zugrunde, eine Vorrichtung zur Zufuhr eines Atemgases zu einem Patienten zu schaffen, durch welche die Beatmungsdruckpegel noch zuverlässiger auf den physiologischen Zustand des Patienten abgestimmt werden können.

Diese Aufgabe wird erfindungsgemäß gelöst durch eine Vorrichtung zur Zufuhr eines Atemgases mit einer elektronischen Signalverarbeitungseinrichtung zur Generierung eines Drucksteuerungssignales auf Grundlage von hinsichtlich der Atmungstätigkeit und/oder des physiologischen Zustandes einer Person indikativen Signalen, wobei die Signalverarbeitungseinrichtung eine Signaleingangseinrichtung und eine Extraktionsehnchtung aufweist, zur Generierung von Datenfeldeinträgen nach Maßgabe vorgegebener Signalanalyseprozeduren, und ein Drucksignalgenerator vorgesehen ist, zur Generierung des Drucksteuerungssignales , wobei der Drucksignalganerator das Drucksignal unter Berücksichtigung wenigstens ausgewählter seitens der Extraktionseinrichtung determiniterter Datenfeldeinträge generiert.

Dadurch wird es auf vorteilhafte Weise möglich, eine auf die momentanen physiologischen Bedürfnisse des Patienten verbessert abgestimmte Atemgasversorgung zu erreichen.

Gem, einer besonders bevorzugten Ausführuugsform der Erfindung ist die Extraktionseinrichtung derart ausgebildet, daß durch diese eine Auswertung von hinsichtlich des Atemgasstromes indikativen Signalen erfolgt Diese hinsichtlich des Atemgasstromes indikativen Signale können beilspielsweise durch eine Staudrukkerfassungseinrichtung oder auch durch Meßschaltungen auf Grundlage akkustischer Prinzipien, insbesondere Dopplereffekt eines Ultraschallereigreignisses, generiert werden.

Die Auswertung der hinsichtlich des Atemgasstromes indikativen Signale erfolgt vorzugsweise auf Grundlage einer Zeitreihenanalyse, insbesondere auf Grundlage einer Varianzbetrachtung der ersten, zweiten und dritten Ableitung des Atemgasstromes.

Vorzugsweise ist die Extraktionseinrichtung derart ausgebildet, daß diese im Zusammenhang mit der Auswertung des Atemgasstromes die Abweichungen des Atemgasstromes von einem Referenzatemstrommuster analysiert. Dieses Referenzatemstrommuster kann kontinuierlich aktualisiert werden.

Es ist möglich, die Extraktionseiniichtung derart auszubilden, daß im Zusammenhang mit der Auswertung der hinsichtlich des Atemgasstromes indikativen Signale ein hinsichtlich des physiologischen Zustandes des Patienten indikativen Klassifikationsdatensatz generiert wird. Dieser Klassifikationsdatensatz kann über eine Schnittstelleneinrichtung, beispielsweise über eine infrarotschnittstelle oder auch durch einen tragbaren Datenträger, einer weiteren Auswertung z.B. durch einen Facharzt, zugeführt werden.

Gem. einer besonders bevorzugten Ausführungsform der Erfindung wird seitens der Extraktionseinrichtung ein Verknüpfungsdatensatz generiert, der einen Zusammenhang zwischen den auf Grundlage ausgewählter Anatysekriterien ermittelten Datenfedeinträgen beschreibt. Dieser Zusammenhang zwischen den extrahierten Merkmaien oder die Wahrscheinlichkeit dieses Zusammenhangs kann durch statistische Angaben weiter klassifiziert werden, so daß die Bedeutung der extrahierten Merkmale, bzw. deren Gewichtung bei der Druckregelung entsprechend berücksichtigt werden Kann.

Es ist möglich, auf Grundlage dieser statistischen Klassifikation der extrahierten Merkmale einen paüentenspezifischen Datensatz zu generleren, aus weichem beispielsweise hervorgeht, daß bestimmte, in den Eingangssignalen enthaltene informationen vernachlässigt werden können. So ist es beispielsweise möglich, anhand des patientenspezifischen Datensatzes zu beurteilen, in weicher Ausprägung ein Zusammenhang zwischen den Beatmungsdruckpegeln und der Körperposition des Patienten, insbesondere dem Haisverdrehungsgrad oder der Kopfposition, besteht. Es ist möglich, mit hoher statistischer Wahrscheinlichkeit auftretende Zusammenhänge statistisch zu beschreiben oder durch Parameter zu definieren.

Gem. einer besonders bevorzugten Ausführungsform der Erfindung ist der Drucksignalgenerator 4 derart operativ in die Signalverarbeitungseinrichtung derart eingebunden, daß dieser bei Generierung des Drucksteuerungssignales P Atemphasendaten sowie Atemphasenumschaltsignale berücksichtigt. Hierdurch wird es möglich, den zeitlichen Verlauf der Atemgasdruckänderung zwischen den abfolgenden Atemphasen derart abzustimmen, daß insbesondere während einer Einschlafphase und hohem Blutsauerstoffgehalt ein besonders hoher Beatmungskomfort gewährleistet ist. Die Atemphasenumschaltsignale werden gem. einer besonders bevorzugten Ausführungsform der Erfindung durch einen Atemphasenkoordinator erzeugt, der derart operativ in die Signalverarbeitungseinrichtung derart eingebunden ist, daß dieser in gleicher Weise wie der Drucksignalgenerator Zugriff auf bestimmte Feldeinträge des durch die Extraktionseinrichtung generierten Datenfeldes hat.

Der Atemphasenkoordinator ist vorzugsweise derart in die Signalvetarbeitungseinrichtung eingebunden, daß dieser auch im wesentlichen in Echtzeit Zugriff auf die an der Signaleingangseinrichtung anliegenden Signale "Rohdaten" oder zumindest davon abgeleiteter (beispielsweise definiert gefilterter) Signale hat. Dadurch wird es möglich, die Atemphasenerkennung unter einer präzisen Abstimmung auf den momentanen physiologischen Zustand des Anwenders vorzunehmen, ohne daß hierbei größere Verzugszeiten bei der Analyse des Atemgasstromes auftreten.

Gem. einer besonders bevorzugten Ausführungsform der Erfindung enfolgt die Atemphesenkoordination durch eine Atemphasenerkennung auf Grundlage einer Schwellwertbetrachtung, wobei der Schweliwert sich mit zunehmendem zeitlichen Abstand vom Zeitpunkt des zurückliegenden Atemphasenwechsels verändert. Diese Änderungsdynamik wird vorzugweise auf Grundlage ausgewählter Einträge in dem Datenfeld programmgesteuert, abgestimmt. Die Atemphasenerkennung erfolgt vorzugsweise ebenfalls unter Rückgriffnahme auf einen umfangreicheren Datensatz, insbesondere ein Kennfeld, wobei die Position innerhalb dieses Kennfeldes in Abhängigkeit von dem momentan erkannten physiologischen Zustand des Anwenders ermittelt wird.

Der Atemphasenkoordinator ist vorzugsweise derart ausgebildet, daß dieser bei der Atemphasenerkennung die erste und vorzugsweise auch die zweite Ableitung des Atemgasstromes analysiert. Eine besonders hohe Zuverlässigkeit bzgl. der Erkennung der einzelnen Atemphasen kann dadurch erreicht werden, daß mehrere Erkennungskriterien verknüpft abgearbeitet werden. Es ist möglich, diese Erkennungskriterien auf Grundlage von FUZZI-Logik Prozeduren auszuwählen oder in Abhängigkeit von den durch die Extraktionseinrichtung generierten Datenfeldeinträgen unerschiedlich zu gewichten.

Der Atemphasenkoordinator und die Druckvorgabeeinrichtung sind vorzugsweise derart operativ verknüpft, daß im Falle unschafer Atemphasenerkennung der Abstand zwischen dem Inspirationsdruck und dem Expirationsdruck verringert wird. Es ist möglich, die Druckvorgabeeinrichtung auch unter Rückgnffnahme auf einen zusätzlichen patientenspezifischen Druckregelungsdatensatz zu betreiben. Der Atemphasenselektionsdatensatz und der Druckregelungsdatensatz können aus Datenfeldern generiert werden, die unter Rückgriffnahme auf die seitens der Extraktionseinrichtung generierten Datenfeldeinträge modifiziert werden.

Gem. einer besonders bevorzugten Ausführungsform der Erfindung ist zur abschließende Einstellung des Atemgasdruckes eine Druckabstimmungseinrichtung vorgesehen, welcher das seitens der Druckvorgabeeinrichtung generierte Drucksteuersignal als Soltgröße zugeführt wird. Die Druckabstimmungseinrichtung übemimmt nach Maßgabe des zugeführten Drucksteuersignales selbsttätig und vorzugsweise mit hoher Regeldynamik die Abstimmung des Atemgasdruckes.

Eine besonders präzise Einstellung des Atemgasdruckes wird hierbei dadurch erreicht, daß der Atemgasdruck an einer patientennahen Druckabgriffstelle erfaßt wird. Es ist möglich, den Atemgasdruck derart einzustellen, daß lediglich der statische Druckanteil an der Druckabgriffstelle, oder auch der statische Druckanteil im Innenraum einer Atemmaske dem nach Maßgabe des Drucksteuersignales vorgegebenen Atemgasdruck entspricht. Soweit ein unmittelbarer Druckabgriff im Bereich eines Anwenders nicht möglich ist, ist gem. einer besonders bevorzugten Ausführungsform der Erfindung die Druckabstimmungseinrichtung derart ausgebildet, daß diese unter Berücksichtigung des momentanen Atemgasstromes Druckkorrekturwerte zur Kompensation leitungsbedingter Druckabfälle errechnet.

Die Erfindungsgemäße Vorrichtung zur Zufuhr eines Atemgases eignet sich in besonderem Maße als CPAP-Gerät mit selbsttätig justierender Bilevel-Druckregelung.

Weitere Ausführungsformen der Erfindung werden im Folgenden beschrieben: <Seite 6a bis 6e>

Weitere Einzelheiten und Merkmale der Erfindung ergeben sich aus der nachfolgenden Beschreibung eines bevorzugten Ausführungsbeispieles in Verbindung mit der Zeichnung. Es zeigen:
Fig. 1 eine Prinzipskizze zur Erläuterung des der Erfindung zugrundeliegenden regelungstechnischen Konzeptes:
Fig. 2 ein mehrdimensionales, patientenspezifisch abgestimmtes Kennfeld, das den Zusammenhang zwischen dem Atemgasdruck und dem hiermit einhergehenden maximalen Atemgasstrom für unterschiedliche physiologische Zustände des Patienten darstellt;
Fig. 3 ein Diagramm zur Erläuterung der in Abhängigkeit von einem Kriterienraster fortlaufend abgestimmten Beurtejiungskriterien zur Erfassung der Atemzyklen eines Patienten sowie zur Abstimmung der Druckumschaltung.

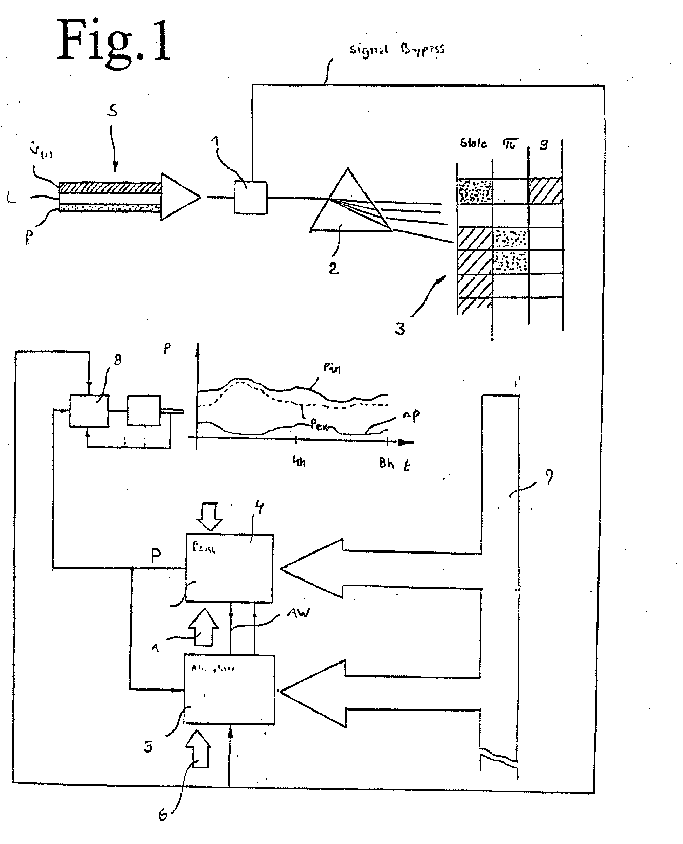

Gem. dem in Fig. 1 veranschaulichten regelungstechnischen Konzept werden hinsichtlich des momentanen physiologischen Zustands eines Patienten indikative Signale einer nachfolgend als Extraktionseinrichtung 2 bezeichneten Verarbeitungseinrichtung zugeführt. In dieser Extraktionseinrichtung 2 wird auf Grundlage einer adaptiven Auswerteprozedur ein Datenfeld 3 ausgefüllt. Die in dem Datenfeld 3 enthaltenen Informationen werden bei der Abstimmung des momentanen Zeitverhaltens der Atemgasdruckregelung berücksichtigt.

Weitere zusätzliche oder alternative Aspekte der Erfindung betreffen die folgenden Punkte.
1. Vorrichtung zur Zufuhr eines Atemgases mit einer elektronischen Signalverarbeitungseinrichtung zur Generierung eines Drucksteuerungssignales (P) auf Grundlage von hinsichtlich der Atmungstätigkeit und/oder des physiologischen Zustandes einer Person indikativen Signalen (S), wobei die Signalverarbeitungseinrichtung eine Signaleingangseinrichtung (1) und eine Extraktionseinrichtung (2) aufweist, zur Generierung von Datenfeldeinträgen (3) nach Maßgabe vorgegebener Signalanalyseprozeduren, und ein Drucksignalgenerator (4) vorgesehen ist, zur Generierung des Drucksteuerungssignales (P), wobei der Drucksignalgenerator (4) das Drucksignal (P) unter Berücksichtigung wenigstens ausgewählter seitens der Extraktionseinrichtung (2) determinierter Datenfeldeinträge (3) generiert.
2. Vorrichtung nach Punkt 1, **dadurch gekennzeichnet, daß** die Extraktionseinrichtung (2) derart ausgebildet ist, daß durch diese eine Auswertung von hinsichtlich des Atemgasstromes indikativen Signalen erfolgt.
3. Vorrichtung nach Punkt 1 oder 2, **dadurch gekennzeichnet, daß** die Extraktionseinrichtung (2) derart ausgebildet ist, daß die Auswertung der hinsichtlich des Atemgasstromes indikativen Signale auf Grundlage einer Zeitreihenanalyse erfolgt.
4. Vorrichtung nach wenigstens einem der Punkte 1 bis 3, **dadurch gekennzeichnet, daß** die Extraktionseinrichtung (2) derart ausgebildet ist, daß im Zusammenhang mit der Auswertung des Atemgasstromes die Abweichungen des Atemgasstromes von einem adaptiv aktualisierten Referenzatemstrommuster analysiert werden.
5. Vorrichtung nach wenigstens einem der Punkte 1 bis 4, **dadurch gekennzeichnet, daß** die Extraktionseinrichtung (2) derart ausgebildet ist, daß im Zusammenhang mit der Auswertung der hinsichtlich des Atemgasstromes indikativen Signale (S) ein hinsichtlich des physiologischen Zustandes des Patienten indikativer Klassifikationsdatensatz generiert wird.
6. Vorrichtung nach wenigstens einem der Punkte 1 bis 5, **dadurch gekennzeichnet, daß** seitens der Extraktionseinrichtung (2) ein Verknüpfungsdatensatz generiert wird, der einen Zusammenhang zwischen extrahierten Merkmalen beschreibt.
7. Vorrichtung nach wenigstens einem der Punkte 1 bis 6, **dadurch gekennzeichnet, daß** unter Zwischenschaltung der Extraktionseinrichtung (2) der Zusammenhang zwischen extrahierten Merkmalen oder die Wahrscheinlichkeit des Zusammenhanges statistisch klassifiziert wird.
8. Vorrichtung nach wenigstens einem der Punkte 1 bis 7, **dadurch gekennzeichnet, daß** unter Zwischenschaltung der Extraktionseinnchtung (2) anhand einer statistischen Klassifikation der extrahierten Merkmals ein patientenspezifischer Datensatz generiert wird.
9. Vorrichtung nach wenigstens einem der. Punkte 1 bis 8, **dadurch gekennzeichnet, daß** der Drucksignalgenerator (4) derart operativ in die Signalverarbeitungseinrichtung eingebunden ist, daß dieser bei der Generierung des Drucksignales (P) Atemphasendaten (A) und Atemphasenumschaltsignale (AW) berücksichtigt.
10. Vorrichtung nach wenigstens einem der Punkte 1 bis 9, **dadurch gekennzeichnet, daß** ein Atemphasenkoordinator (5) vorgesehen ist, zur Generierung von Atemphasendaten (A).
11. Vorrichtung nach wenigstens einem der Punkte 1 bis 10, **dadurch gekennzeichnet, daß** der Atemphasenkoordinator (5) operativ in die Signalverarbeitungseinrichtung derart eingebunden ist, daß dieser auf die Datenfeldeinträge (3) Zugriff hat.
12. Vorrichtung nach wenigstens einem der, Punkte, 1 bis 11, **dadurch gekennzeichnet, daß** der Atemphasenkoordinator (5)) operativ in die Signalverarbeitungseinrichtung derart eingebunden ist, daß dieser auf wenigstens einen Teil der an der Signaleingangseinrichtung (1) anliegenden Signale - oder davon abgeleiteter Signale Zugriff hat.
13. Vorrichtung nach wenigstens einem der Punkte 1 bis 12, **dadurch gekennzeichnet, daß** der Atemphasenkoordinator (5)) operativ in die Signalverarbeitungseinrichtung derart eingebunden ist, daß dieser auf einen patientenspezifischen Atemphasenselektionsdatensatz (6) Zugriff hat.
14. Vorrichtung nach wenigstens einem der Punkte 1 bis 13, **dadurch gekennzeichnet, daß** der Atemphasenkoordniator (5) derart ausgebildet ist, daß dieser eine Atemphasenerkennung auf Grundlage sich zeitlich ändernder Kriterien vornimmt.
15. Vorrichtung nach wenigstens einem der Punkte 1 bis 13, **dadurch gekennzeichnet, daß** der Atemphasenkoofdinator (5) eine Atemphasenerkennung in Verbindung mit einer Schwettwertbetrachtung vornimmt, wobei der Schwellwert sich mit zunehmendem zeitlichen Abstand von dem Zeitpunkt des zurückliegenden Alemphasenwachsel verändert.
16. Vorrichtung nach wenigstens einem der. Punkte 1 bis 15, **dadurch gekennzeichnet, daß** der Atemphasenkoordinator (5) derart ausgebildet ist, daß der Schweliwert derart verändert wird, daß die Umschaltsensibilität zunimmt.
17. Vorrichtung nach wenigstens einem der Punkte 1 bis 16, **dadurch gekennzeichnet, daß** der Atemphasenkoordinator (5) derart ausgebildet ist, daß zur Alemphasenerkennung die erste und/oder die zweite Ableitung des Atemgasstromes analysiert wird.
18. Vorrichtung nach wenigstens einem der Punkte 1 bis 17, **dadurch gekennzeichnet, daß** der Atemphasenkoordinator (5) derart ausgebildet ist, daß im Rahmen der Atemphasenerkennung mehrere Erkennungskriterien verknüpft betrachtet werden.
19. Vorrichtung nach wenigstens einem der Punkte 1 bis 18, **dadurch gekennzeichnet, daß** der Atemphasenkoordinator (5) und der Drucksignatgenerator (4) derart miteinander operativ verknüpft sind, daß im Falle unscharfer Atemphasenerkennung der Abstand zwischen dem Inspirationsdruck und dem Expirationsdruck verringert wird.
20. Vorrichtung nach wenigstens einem der Punkte 1 bis 19, **dadurch gekennzeichnet, daß** der Drucksignalgenerator (4) auf einen patientenspezifischen Druckregelungsdatensatz (7) Zugriff hat.
21. Vorrichtung nach wenigstens einem der Punkte 1 bis 20, **dadurch gekennzeichnet, daß** der Atemphasenselektionsdatensatz (6) und/oder der Druckregelungsdatensatz (7) unter Rückgriffnahme auf die seitens der Extraktionseinrichtung (2) generierten Datenfeldeinträge (3) generiert werden.
22. Vorrichtung nach wenigstens einem der. Punkte 1 bis 21, **dadurch gekennzeichnet, daß** eine Druckabstimmungseinrichtung (8) vorgesehen ist, zur Einstellung des Atemgasdruckes nach Maßgabe des Drucksteuersignales.
23. Vorrichtung nach wenigstens einem der Punkte 1 bis 21, **dadurch gekennzeichnet, daß** die Druckabstimmungseinrichtung (8) den Atemgasdruck an einer patientennahen Druckabgriffsstelle erfaßt.
24. Vorrichtung nach wenigstens einem der Punkte 1 bis 23, **dadurch gekennzeichnet, daß** die Druckabstimmungseinrichtung (8) den Atemgasdruck derart abstimmt, daß dessen statischer Druckanteil an der Druckabgriffsstelle oder im Innenraum einer Atemmaske dem nach Maßgabe des Drucksteuersignales (P) vorgegebenen Atemgasdruck entspricht.
25. Vorrichtung nach wenigstens einem der Punkte 1 bis 24, **dadurch gekennzeichnet, daß** die Druckabstimmungseinrichtung (8) eine Druckkorrektureinrichtung umfaßt, zur Kompensation leitungsbedingter Druckabfälle.
26. CPAP-Gerät mit einer sich selbst auf die physiologischen Bedürfnisse eines Patienten abstimmenden Bilevel-Druckregelung bei weichem hinsichtlich des Atemgasstromes indikative Signale durch eine Extraktionseinrichtung analysiert werden und hierauf basierende Einträge in einem Datenspeicher vorgenommen werden, wobei ein Drucksignalgenerator und eine mit diesem operativ verbundene Atemphasenerkennungseinrichtung vorgesehen ist, und das Regelverhalten des Drucksignalgenerators sowie der Atemphasenerkennungseinrichtung auf Grundiage der Einträge in dem Datenspeicher dynamisch veränderbar sind.

Bei dem hier gezeigten System wird ein Teil der Feldeinträge bei der Ermittiung eines Soll-inspirationsdruckes sowie eines Soll-Expirationsdruckes berücksichtigt.

Diese Solldrücke werden in einem Atemphasenkoordinator 5 in Verbindung mit weiteren aus dem Kriterienfeld 3 ausgelesenen Einträgen verarbeitet. Der derart festgelegte momentane Atemgasdruckwert wird einer Drucksteuereinrichtung 8 zugeführt, die in Verbindung. mit einem hochdynamischen Regelkreis den Atemgasdruck, beispielsweise durch Steuerung einer Gebläsedrehzahl auf den vorgegebenen Atemgassolidruck einstellt. Die extrem zeitnahe Elnstellung der Atemgasdrücke wird bei dem gezeigten Regelungskonzept erreicht, indem zumindest ein Teil der patientenseitig abgegriffenen Signale S lediglich durch einen Bandpaß gefiltert, den einzeinen Regelungskomponenten zur Verfügung steht.

Das erfindungsgemäße Regelungskonzept kann durch eine ausreichend leistungsfähige Rechnereinrichtung realisiert werden, wobei es möglich ist, die hier klar abgegrenzt beschriebenen Komponenten in der Gestalt sich ggf. überlagernder Unterprozeduren durch einen entsprechenden Programmablauf zu verwirkilchen.

In der Extraktionseinrichtung 2 wird vorzugsweise wenigstens ein hinsichtlich des momentanen Atemgasstromes indikatives Signal einer Zeitrelhenanalyse unterzogen, wobei vorzugsweise wenigstens die erste, die zweite und die dritte Ableitung dieses Signals gebildet werden. Weiterhin werden statistische Parameter, insbesondere die Varianz des Slgnalveriaufes, bzw. dessen zeitliche Ableitungen ermittelt. Die im Zusammenhang mit den einzelnen Kurvendiskussionskriterien ermittelten Merkmale werden in dem Feld 3 vorzugsweise in Verbindung mit einer statistischen Sicherheit sowie einem sich ggf. mit zunehmender statistischer Aussagefähigkeit ändernden Gewichtungsfaktor oder Verknüpfungsparametem, eingetragen. Zumindest ein Teil der in das Feld 3 eingetragenen Angaben wird bei der Abstimmung des Zeitverhaltens der eigentlichen Atemgasdruckregelung berücksichtigt.

Bei dem hier gezeigten Ausführungsbeispiel werden über die Signaieingangseinrichtung 1 neben bzgl. des Atemflusses indikativen Signalen auch Lagesignale sowie hinsichtlich des Atemgasdruckes indikativen Signale erfaßt. Diese Signale stehen in definiert gefilterter Form bedarfsweise den regelungstechnischen Systemen der erfindungsgemäßen Signalverarbeitungseinrichtung zur Verfügung. Durch die Extraktionseinrichtung 2 wird bei der hier dargestellten Ausführungsform ein mehrdimensionales Datenfeld 3 erzeugt. In diesem Datenfeld 3 befinden sich die sich dynamisch ändernden, durch die Extraktionseinrichtung generierten Datenfeldeinträge. Bei der gezeigten Ausführungsform werden bestimmten, durch die Extraktionseinrichtung 2 generierten Datenfeldeinträgen weitere Angaben zugeordnet, insbesondere Angaben zur statistischen Sicherheit. Die in dem nach Maßgabe der Extraktionseinrichtung 2 dynamisch verwalteten Datenfeld 3 abgelegten Datenfeldeinträge stehen, wie hier durch Datenfiußpfeile 9 angedeutet, der Druckvorgabeeinrichtung 4 sowie dem Atemphasenkoordinator 5 zur Verfügung.

Die Druckvorgabeeinrichtung 4 beispielsweise ermittelt auf Grundlage, ausgewählter Datenfaldeinträge in dem dynamischen Datenfeld 3 den maximalen Inspirationsdruck sowie den maximalen Expirationsdmck. Weiterhin ermittelt die Druckvorgabeeinrichtung 4 einen; auf den momentanen physiologischen Zustand der spontan atmenden Person abgestimmten zeitlichen Druckverlauf beim Wechsel zwischen den beiden Atemphasen.

Die Erkennung der Atemphasen erfolgt über den Atemphasenkoordinator 5, der ebenfalls Zugriff auf ausgewählte Datertfefdemträge in dem Datenfeld 3 hat. Der Atemphasenkoordinator 5 steht weiterhin mit der Signaleingangseinrichtung 1 in einem nahezu totzeitfreien Signalverbund. Die Beurteilung der Atmungstätigkeit der spontan atmenden Person durch den Atemphasenkoordinator 5 erfolgt bei dem dargestellten Ausführungsbeispiel zusätzlich unter Berücksichtigung patientenspezifischer Vorgabegrößen sowie auch unter Berücksichtigung der seitens der Druckvorgabeeinrichtung 4 vorgegebenen Drucksteuerungssignale P

Hierdurch wird es möglich, bei Atemgaszufuhrperioden mit vergleichsweise großen Unterschieden zwischen dem Inspirationsatemgasdruck und dem Expirationsatemgasdruck die Atemphasenerkennung mit höherer Sensibilität vorzunenmen, wogegen bei vergleichsweise kleinen Differenzen zwischen dem Atemgasinspirationsdruck und dem Atemgasexpirationsdruck andere Kriterien der Erkennung bzw. der Bestimmung der Atemphasen zugrunde gelegt werden können.

Die seitens der Druckvorgabeeinrichtung 4 generierten Drucksteuerungssignale P werden einer Druckabstimmungseinrichtung 8 zugeführt, durch welche die für den Aufbau des Atemgasdruckes relevanten Organe, beispielsweise eine Gebläseeinrichtung, angesteuert werden. Die Reglung des Atemgasdruckes nach Maßgabe des Drucksteuerungssignales P kann über einen hierzu vorgesehenen hochdynamischen Regelkreis mit eigenen Druckerfassungsorganen erfolgen.

Im Zusammenspiel mit der beschriebenen, erfindungsgemäßen Signalverarbeitungseinrichtung wird es möglich, eine stabile und präzise auf die physiologischen Bedürfnisse einer spontan atmenden Person abgestimmte Einstellung des Atemgasdruckes zu gewährleisten.

Wie aus dem, in die Darstellung nach Fig. 1 eingefügten Diagramm hervorgeht, variieren der mittlere inspiratbnsdruck und der mittlere Expirationsdruck einer Atemphasen im Laufe einer sich über die ganze Schlafphase eines Patienten erstreckenden Zeitperiode. In Abhängigkeit von bestimmten, hinslchtlich des physiologischen Zustands der spontan atmenden Person indikativen Größen ergeben sich hierbei unterschiedlich große Druckabstande (Δp) zwischen dem Inspirationsdruck und dem mittleren Explrationsdruck jedes Aternzuges. In Abhängigkeit von dieser Druckdifferenz wird auch die Erkennung der Atemphasen durch den Atemphasenselektor 5 abgestimmt.

Die Abstimmung sowohl des Inspirationsdruckes als auch des Expirationsdruckes kann auf Grundlage eines patientenspezifisch adaptierten Kennfelder erfolgen, wobei die momentane Position in diesem Kennfeld auf Grundlage von Klassifikationsangaben erfolgen kann, die im Zusammenspiel mit den durch die Extraktionseinrichtung 2 generierten Datenfeldeinträgen 3 ermittelt werden.

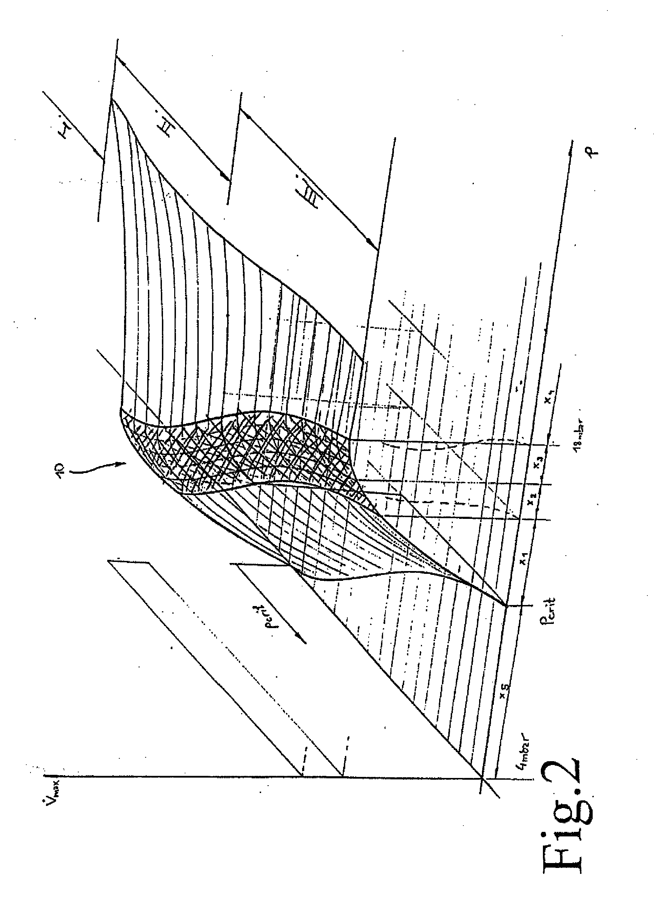

Bei dem in Fig. 2 dargestellten Ausführungsbeispiel wird anhand der in dem dynamischen Datenfeld 3 vorhandenen Datenfeldeinträge ermittelt, weicher Bereich des hier gezeigten Kennfeldes für die momentane Regelung des Atemgasdruckes heranzuziehen ist. Auf Grundlage einer derart getroffenen Auswahl des patientenspezifisch angepaßten Kennfeldbereiches wird ein maximaler Atemoasinspirationsdruck sowie hier zugehörig ein minimaler Atemgasexpirationsdruck ermittelt. Diese beiden Druckpegel liegen vorzugsweise innerhalb des hier stärker schraffiert angedeuteten Kniebereiches 10 des Kennfeldes. Im vorliegenden Beispiel erstreckt sich das Kennfeld über einen CPAP-Druckbereich von 4 - 18 mb. Der Abstand zwischen dem maximalen Inspirationsdruck dem minimalen Expirationsdruck liegt bei dem hier gezeigten patientenspezifischen Kennfeld unterhalb von 6 mb. Der Abstand zwischen dem maximalen Inspirationsdruck und dem minimalen Expirationsdruck wird neben der Steigung des Kennfeldes in dem dargestellten Kniebereich 10 auch durch weitere Einflußfaktoren bestimmt, die auf Grundlage des durch die Extraktionseinrichtung 2 dynamisch aufgefüllten Datenfeldes ermittelt werden.

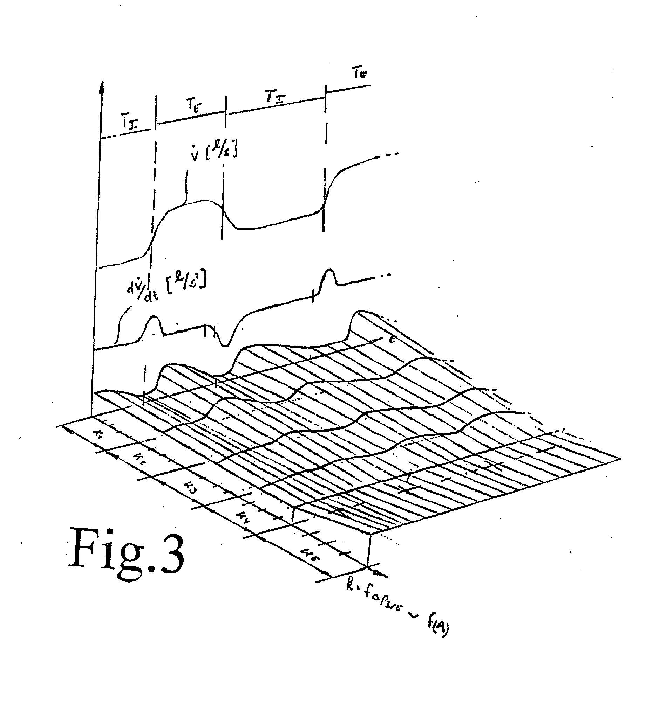

Die Darstellung gem. Fig. 3 zeigt qualitativ den zeitlichen Verlauf des Atemgasstromes V unter Zuordnung des seitens des Atemphasenkoordinators 5 erkannten Inspirationsphasen T₁ und Expirationsphasen T_{E}. Unterhalb der Atemgasstromkurve ist die erste Ableitung derselben skizziert. In Abhängigkeit von den Einträgen in dem Datenfeld 3 sowie vorzugsweise auch in Abhängigkeit von den seitens der Druckvorgabeehnchtung 4 vorgegebenen Drucksteuerungssignal P werden die Kriterien für die Atemphasenerkennung abgestimmt. Bei dem hier gezeigten Ausführungsbeispiel erfolgt in einer ersten Atemphasenklassifikationskategorie K₁ die Atemphasenerkennung durch einen Schwellwertvergleich mit einem sich zeitlich mit hoher Dynamik von einem vergleichsweise großen Wert zu einem relativ niedrigen Wert ändernden Schwellwert. Unmittelbar nach der Erkennung eines Atemphasenwechsels wird der Schwellwert wieder erhöht. Die unmittelbare Zurückschaltung in die vorangegangene Atemphase wird durch eine dynamisch angepaßte Totzeit vermieden. In der Klassifikationskategorie K₂ ist die Abweichung zwischen dem maximalen Schwellwert und dem minimalen Schwellwert bereits geringer. Noch geringer ist der Unterschied zwischen den beiden Schwerwerten in der Klassifikationskategorie 3. In der Klassifikationskategorie K₄ erfolgt die Atemphasenerkennung auf Grundlage eines nahezu konstanten Schwellwertes. In der Klassifikationskategorie K₅ erfolgt ebenfalls eine Atemphasenerkennung auf Grundlage konstanter, jedoch in Abhängigkelt von den Datenfeideinträgen zunehmenden Schwellwerlen.

Die Klassifikationskategorien werden vorzugsweise in Abhängigkeit von einer durch die Extraktionseinrichtung 2 durchgeführten und durch Datenfeldeinträge kommunizieren, Atemmustererkennung durchgeführt.

Durch die erfindungsgemäße Vorrichtung wird es möglich, ein CPAP-Gerät zur Durchführung einer Atemtherapie im häuslichen Bereich zu schaffen, das den Atemgasdruck sowohl für den inspirationsvorgang als auch für den Expirationsvorgang selbsttätig auf die physiologischen Bedürfnisse eines Anwenders abstimmt.

Durch die erfindungsgemäß vorgesehene Extraktionseinrichtung wird es möglich, den momentanen physiologischen Zustand des Anwenders durch Erfassung der Atmungstätigkeit zu klassifizieren. Auf Grundlage dieser Klassifikation wird es möglich, die momentan günstigsten Atemgasdruckwerte für den inspirationsvorgang und für den Expirationsvorgang festzuiegen. Weiterhin wird es möglich, die für die Einstellung der ggf. abweichenden Druckregel erforderliche Atemphasenerkennung mit hoher Präzision durchzuführen.

## Patentansprüche

1. Vorrichtung zur Zufuhr eines Atemgases zu einem Patienten, mit einer Signalverarbeitungseinrichtung, die eine Signaleingangseinrichtung (1) und eine Extraktionseinrichtung (2) aufweist,
wobei die Signaleingangseinrichtung (1) eingerichtet ist zum Empfangen von hinsichtlich eines Atemgasflusses indikativen Signalen, von Lagesignalen des Patienten und von hinsichtlich des Atemgasdrucks indikativen Signalen, und wobei die Extraktionseinrichtung (2) zur dynamischen Generierung eines mehrdimensionalen Datenfelds (3) nach Maßgabe vorgegebener Signalanalyseprozeduren eingerichtet ist, wobei das Datenfeld (3) den Zusammenhang zwischen dem Atemgasdruck und dem hiermit einhergehenden maximalen Atemgasstrom für unterschiedliche physiologische Zustände des Patienten darstellt,
einem Drucksignalgenerator (4) der ausgebildet ist, auf Grundlage ausgewählter Datenfeldeinträge in dem dynamischen Datenfeld (3), einen maximalen Inspirationsdruck, einen maximalen Expirationsdruck, und einen auf den momentanen physiologischen Zustand des Patienten abgestimmten zeitlichen Druckverlauf beim Wechsel zwischen den beiden Atemphasen, zu ermitteln, wobei der Drucksignalgenerator weiterhin eingestellt ist, ein Drucksteuersignal (P) zu generieren, das insbesondere für den auf den momentanen physiologischen Zustand des Patienten abgestimmten zeitlichen Druckverlauf beim Wechsel zwischen den beiden Atemphasen indikativ ist, einem Atemphasenkoordinator (5) zur Generierung von Atemphasendaten (A) auf der Basis von Datenfeldeinträgen des mehrdimensionalen Datenfelds (3) und auf Basis der seitens der Druckvorgabeeinrichtung (4) vorgegebener Drucksteuerungssignale (P),
eine Druckabstimmungseinrichtung (8), zum Empfangen des seitens der Druckvorgabeeinrichtung (4) generierten Drucksteuerungssignals (P) und zur Einstellung des Atemgasdruckes nach Maßgabe des Drucksteuersignals.

2. Vorrichtung nach Anspruch 1, ferner aufweisend einen Signal Bypass der ausgebildet ist, die von der Signaleingangseinrichtung empfangenen Signale vorbeizuführen, vorzugsweise der Druckabstimmungseinrichtung (8) direkt zuzuführen.

3. Vorrichtung nach Anspruch 2, ferner aufweisend ein Filter, vorzugsweise einen Bandpass, zum filtern der Patientenseitig abgegriffenen Signale (S).

4. Vorrichtung nach einem der vorangehenden Ansprüche, wobei die Signalverarbeitungseinrichtung ausgebildet ist, auf der Basis einer statistischen Klassifikation der Einträge des patientenspezifischen Datenfelds, einen patientenspezifischen Datensatz zu generieren, der einen Zusammenhang zwischen den Beatmungsdruckpegeln und einem physiologischen Patientenzustand, bevorzugt der Körperposition des Patienten und insbesondere dem Halsverdrehungsgrad oder der Kopfposition, definiert.

5. Vorrichtung nach einem der vorangehenden Ansprüche, wobei die hinsichtlich des Atemgasstromes indikativen Signale durch eine Staudrukkerfassungseinrichtung generiert werden.

6. Vorrichtung nach einem der vorangehenden Ansprüche, wobei die hinsichtlich des Atemgasstromes indikativen Signale durch Messschaltungen auf Grundlage akkustischer Prinzipien, insbesondere des Dopplereffekt eines Ultraschallereignisses, generiert werden.

7. Vorrichtung nach einem der vorangehenden Ansprüche, wobei der Atemphasenkoordinator (5) mit der Signaleingangseinrichtung (1) in einem nahezu totzeitfreien Signalverbund steht.

8. Vorrichtung nach einem der vorangehenden Ansprüche, wobei der Atemphasenkoordinator (5) ausgebildet ist, die Atemphase in Abhängigkeit der Differenz zwischen dem Inspirationsdruck und dem mittleren Expirationsdruck über einen Zeitabschnitt, vorzugsweise jedes Atemzuges, und des physiologischen Zustands des Patienten zu erkennen.

9. Vorrichtung nach einem der vorangehenden Ansprüche, wobei das patientenspezifische Kennfeld den maximalen Atemgasinspirationsdruck sowie den minimalen Atemgasexpirationsdruck des Patienten umfasst.

10. Vorrichtung nach einem der vorangehenden Ansprüche, wobei der maximale Atemgasinspirationsdruck sowie der minimale Atemgasexpirationsdruck in einem Bereich von 4-18 mb liegen.

11. Vorrichtung nach einem der vorangehenden Ansprüche, wobei der Atemphasenkoordinator (5) derart ausgebildet ist, dass zur Atemphasenerkennung die erste und/oder die zweite Ableitung des Atemgasstromes analysiert wird.

12. Vorrichtung nach einem der vorangehenden Ansprüche, wobei der physiologische Zustand des Patienten momentane physiologische Bedürfnisse des Patienten umfasst.

13. Vorrichtung nach Anspruch 12, wobei die momentanen physiologischen Bedürfnisse des Patienten den Blutsauerstoffgehalt während der Einschlafphase umfassen.

14. CPAP-Gerät mit einem Gebläse zum generieren eines Atemgasstroms und einer Vorrichtung nach einem der vorangehenden Ansprüche.

15. CPAP-Gerät nach Anspruch 14 mit selbstabstimmender Bilevel-Druckregelung.
